# EUROPEAN PATENT APPLICATION

(11) **EP 3 888 750 A1**
(43) Date of publication of application: **06.10.2021**
(21) Application number: 20167682.2
(22) Date of filing: 02.04.2020
(51) Int. Cl.: A61P 7/06, C07D 401/04, A61K 31/4439

(54) **CRYSTALLINE FORM OF VOXELOTOR**

(71) Applicant: Sandoz AG, 4056 Basel (CH)
(72) Inventor: MARTIN, Nolwenn, 6250 Kundl (AT); SCHWARZ, Franz Xaver, 6250 Kundl (AT)
(74) Representative: Kluschanzoff, Harald

(57) **Abstract**

The present invention relates to a crystalline form of voxelotor. Furthermore, the invention relates to a pharmaceutical composition comprising said crystalline form of voxelotor and at least one pharmaceutically acceptable excipient. The pharmaceutical composition of the present invention can be used as a medicament, in particular for the treatment of sickle cell disease.

## Description

### FIELD OF THE INVENTION

The present invention relates to a crystalline form of voxelotor. Furthermore, the invention relates to a pharmaceutical composition comprising said crystalline form of voxelotor and at least one pharmaceutically acceptable excipient. The pharmaceutical composition of the present invention can be used as a medicament, in particular for the treatment of sickle cell disease.

### BACKGROUND OF THE INVENTION

Voxelotor is also known under the chemical name 2-hydroxy-6-((2-(1-isopropyl-1*H*-pyrazol-5-yl)pyridin-3-yl)methoxy)benzaldehyde and can be represented by the chemical structure as depicted in Formula (I)

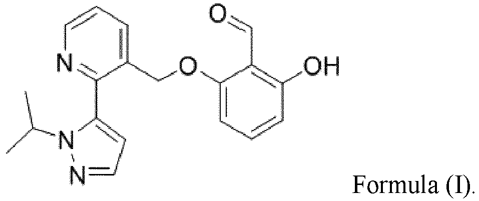

Voxelotor is a hemoglobin S (HbS) polymerization inhibitor indicated for the treatment of sickle cell disease. Upon administration, voxelotor targets and covalently binds to the *N-*terminal valine of the alpha chain of HbS. This stabilizes HbS, thereby improving oxygen binding affinity. The binding of voxelotor to HbS prevents HbS polymerization, reduces sickling, decreases red blood cell (RBC) damage and increases the half-life of RBCs. As a consequence, blood flow is improved and hemolytic anemia is decreased.

WO 2013/102142 A1 discloses substituted benzaldehyde compounds used for increasing tissues oxygenation, including voxelotor (compound 43). Examples 17 and 18 describe the synthesis of voxelotor, which is isolated after chromatographic purification as a pale yellow oil or a pale yellow solid, respectively, and is characterized by nuclear magnetic resonance (NMR) spectroscopy and mass spectrometry. No further data regarding solid state are given.

WO 2015/120133 A1 discloses crystalline forms of voxelotor, in particular the ansolvate forms designated as Form I, Form II and Material N as well as several solvates.

According to WO 2015/120133 A1 Form I is a metastable phase, which generally nucleates first from a slurry and transforms to free base Form II, a thermodynamically more stable phase relative to Form I, by extending the slurry time. Free base Material N is reported in WO 2015/120133 A1 as being stable relative to Form I and Form II at room temperature and is enantiotropically related to Form II with a specific transition temperature estimated to be near 40°C. At operating temperatures above this transition temperature, i.e. above 40°C (*e.g.* at 50°C) free base Form II appears to be the most stable form, relative to Form I and Material N, and voxelotor exists primarily as Form II, which may contain some residual Material N. Under operating temperatures below this transition temperature, i.e. below 40°C (e.g. at 30°C) voxelotor exists primarily as Material N, which may contain some residual Form II.

Different solid-state forms of an active pharmaceutical ingredient (API) often possess different physical and chemical properties such as, but not limited to, dissolution rate, solubility, chemical stability, physical stability, hygroscopicity, melting point, morphology, flowability, wettability, bulk density and compressibility. It is therefore of utmost importance to avoid any uncontrolled polymorphic transitions which may happen during drug product manufacturing and/or storage, since any change in the solid state of the API may compromise the quality and safety of the final drug product containing the API.

The described solid-state interconversion of Form I, Form II and Material N are critical, since these transformations may also occur during pharmaceutical processing and/or storage, thus leading to non-uniform drug products.

Hence, there remains a need for the provision of an improved solid state form of voxelotor, in particular a form which can be reliably produced in pure polymorphic form and which preserves its crystal structure during drug product manufacturing and storage of the drug product throughout the whole shelf-life.

### SUMMARY OF THE INVENTION

The present invention provides a new crystalline form of voxelotor, which possesses one or more improved physicochemical properties selected from the group consisting of dissolution rate, solubility, wettability, chemical stability, physical stability, polymorphic purity, chemical purity, hygroscopicity, morphology, flowability, bulk density and compressibility.

In particular, the crystalline form of voxelotor of the present invention is a phase pure highly crystalline form of voxelotor with excellent physicochemical stability during pharmaceutical processing and storage and therefore allows for the preparation of a uniform and thus safe and efficacious drug product.

### Abbreviations

- PXRD: powder X-ray diffractogram
- DSC: differential scanning calorimetry
- w-%: weight percent
- API: active pharmaceutical ingredient

### Definitions

In the context of the present invention the following definitions have the indicated meaning, unless explicitly stated otherwise:
As used herein the term "room temperature" refers to a temperature in the range of from 20 to 30°C.

As used herein, the term "measured at a temperature in the range of from 20 to 30°C" refers to a measurement under standard conditions. Typically, standard conditions mean a temperature in the range of from 20 to 30°C, *i.e.* at room temperature. Standard conditions can mean a temperature of about 22°C. Typically, standard conditions can additionally mean a measurement under 20-50% relative humidity.

The term "reflection" with regard to powder X-ray diffraction as used herein, means peaks in an X-ray diffractogram, which are caused at certain diffraction angles (Bragg angles) by constructive interference from X-rays scattered by parallel planes of atoms in solid material, which are distributed in an ordered and repetitive pattern in a long-range positional order. Such a solid material is classified as crystalline material, whereas amorphous material is defined as solid material, which lacks long-range order and only displays short-range order, thus resulting in broad scattering. According to literature, long-range order e.g. extends over approximately 100 to 1000 atoms, whereas short-range order is over a few atoms only (see "Fundamentals of Powder Diffraction and Structural Characterization of Materials" by Vitalij K. Pecharsky and Peter Y. Zavalij, Kluwer Academic Publishers, 2003, page 3).

The term "essentially the same" with reference to powder X-ray diffraction means that variabilities in reflection positions and relative intensities of the reflections are to be taken into account. For example, a typical precision of the 2-Theta values is in the range of ± 0.2° 2-Theta, preferably in the range of ± 0.1° 2-Theta. Thus, a reflection that usually appears at 8.8° 2-Theta for example can appear between 8.6° and 9.0° 2-Theta, preferably between 8.7 and 8.9° 2-Theta on most X-ray diffractometers under standard conditions. Furthermore, one skilled in the art will appreciate that relative reflection intensities will show inter-apparatus variability as well as variability due to degree of crystallinity, preferred orientation, sample preparation and other factors known to those skilled in the art and should be taken as qualitative measure only.

The term "solid-state form" as used herein refers to any crystalline and/or amorphous phase of a compound. Crystalline phases include anhydrous/non-solvated forms of a compound and their polymorphs, hydrates and solvates of a compound and their polymorphs, salts and co-crystals of a compound and their polymorphs and pseudopolymorphic forms and any mixtures thereof.

The terms "non-solvated" or "ansolvate" which may be used herein interchangeably, when talking about a crystalline solid indicates that no organic solvent is cooperated in or accommodated by the crystal structure. Non-solvated forms may still contain residual organic solvents, which are not part of the crystal structure but may be adsorbed on the surface or absorbed in disordered regions of the crystal.

The term "solvate" as used herein, refers to a crystalline solid were either one or more organic solvent(s) is/are cooperated in or accommodated by the crystal structure e.g. is/are part of the crystal structure or entrapped into the crystal (solvent inclusions). Thereby, the one or more organic solvent(s) can be present in a stoichiometric or non-stoichiometric amount. When the one or more organic solvent(s) is/are present in stoichiometric amount(s), the solvate may be referred to by adding greek numeral prefixes. For example, a solvate may be referred to as a *hemi*solvate or as a *mono*solvate depending on the solvent(s)/compound stoichiometry. The solvent content can be measured, for example, by gas chromatography, NMR spectroscopy, single-crystal X-ray diffraction and/or thermogravimetric analysis-mass spectrometry.

As used herein, the term "essentially free of any other solid-state form" with reference to the composition comprising the crystalline form of voxelotor of the present invention, means that the composition contains at most 20 w-%, preferably at most 10 w-%, more preferably at most 5 w-%, 4 w-%, 3 w-%, 2 w-% or 1 w-% of any other solid-state form of voxelotor, based on the total weight of the composition.

The crystalline form of voxelotor of the present invention may be referred to herein as being characterized by a powder X-ray diffractogram "as shown in" a figure. The person skilled in the art understands that factors such as variations in instrument type, response and variations in sample directionality, sample concentration, sample purity, sample history and sample preparation may lead to variations, for example relating to the exact reflection positions and intensities. However, a comparison of the graphical data in the figure herein with the graphical data generated for an unknown physical form and the confirmation that two sets of graphical data relate to the same crystal form is well within the knowledge of a person skilled in the art.

A "predetermined amount" as used herein with regard to the crystalline form of voxelotor of the present invention refers to the initial amount of the crystalline form of voxelotor of the present invention used for the preparation of a pharmaceutical composition having a desired dosage strength of voxelotor.

As used herein, the term "effective amount" in conjunction with the crystalline form of voxelotor of the present invention encompasses an amount of the crystalline form of voxelotor of the present invention which causes the desired therapeutic or prophylactic effect.

The term "pharmaceutically acceptable excipient" as used herein refers to substances, which do not show a significant pharmacological activity at the given dose and that are added to a pharmaceutical composition in addition to the API. Excipients may take the function of vehicle, diluent, release agent, disintegrating agent, dissolution modifying agent, wetting agent, absorption enhancer, stabilizer or a manufacturing aid among others. Excipients may include fillers (diluents), binders, disintegrants, surfactants, lubricants and glidants.

### BRIEF DESCRIPTION OF THE FIGURES

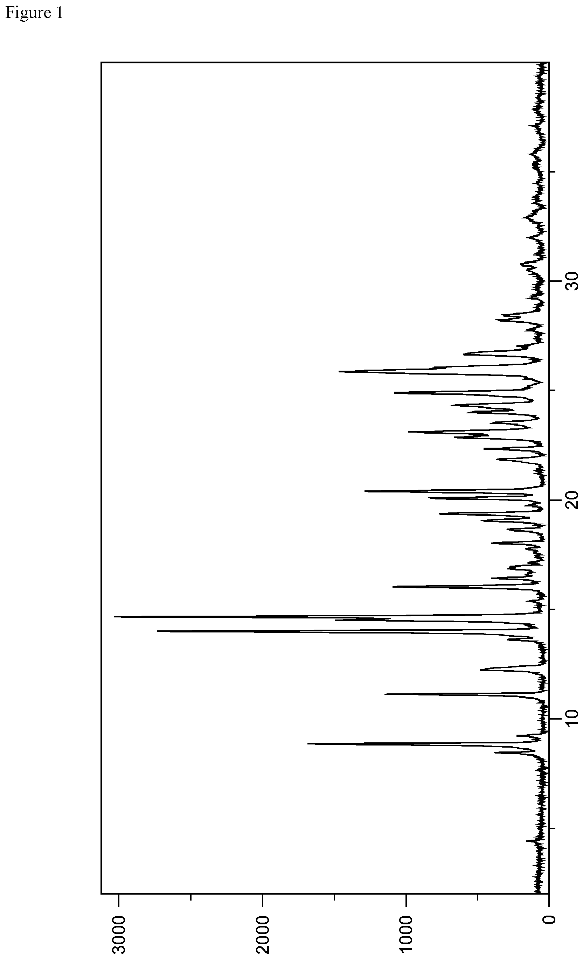

**Figure 1****:** illustrates a representative PXRD of the crystalline form of voxelotor according to the present invention. The x-axis shows the scattering angle in °2-Theta, the y-axis shows the intensity of the scattered X-ray beam in counts of detected photons.

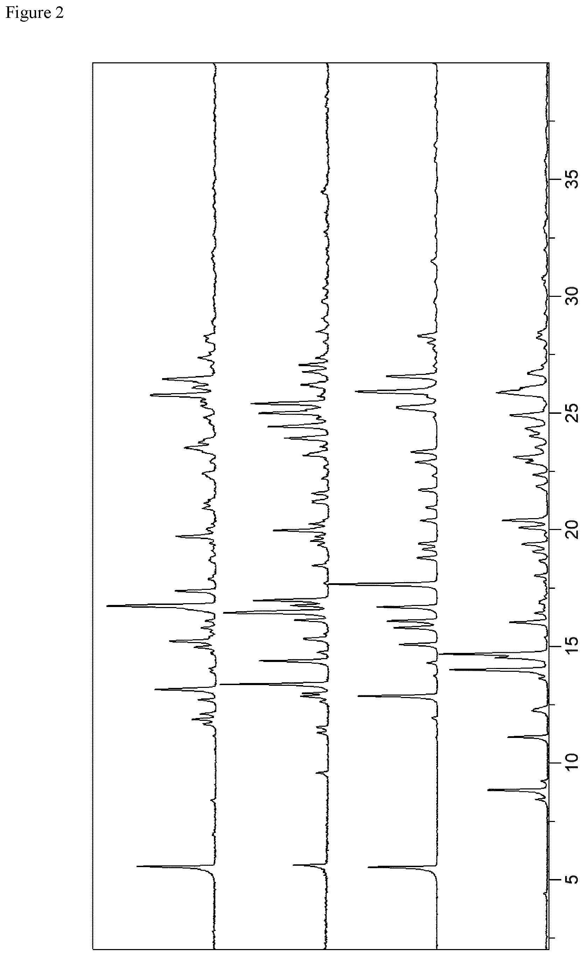

**Figure 2****:** illustrates a comparison of representative PXRDs (from bottom to top) of the crystalline form of voxelotor of the present invention, Form I, Form II and Material N of WO 2015/120133 A1, respectively. The x-axis shows the scattering angle in °2-Theta. The powder X-ray diffractograms of Form I, Form II and Material N were shifted along the y-axis to separate the diffractograms for clarity. Intensities were adapted so that the most intense reflections of the PXRDs were uniform. Thus, no intensity scale is shown on the linear vertical axis.

### DETAILED DESCRIPTION OF THE INVENTION

The present invention relates to a crystalline form of voxelotor.

The crystalline form of voxelotor of the present invention may be characterized by analytical methods well known in the field of the pharmaceutical industry for characterizing crystalline solids. Such methods comprise but are not limited to powder X-ray diffraction, differential scanning calorimetry and thermogravimetric analysis. The crystalline form of voxelotor of the present invention may be characterized by one of the aforementioned analytical methods or by combining two or more of them. In particular, the crystalline form of voxelotor of the present invention may be characterized by any one of the following embodiments or by combining two or more of the following embodiments.

In a first aspect, the invention relates to a crystalline form of voxelotor having the chemical structure as depicted in Formula (I) 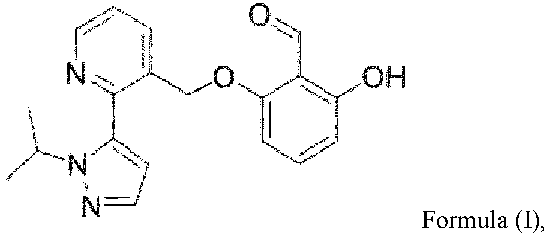 characterized by having a PXRD comprising reflections at 2-Theta angles of:
(4.4 ± 0.2)°, (8.8 ± 0.2)° and (14.7 ± 0.2)°; or
(4.4 ± 0.2)°, (8.8 ± 0.2)°, (12.2 ± 0.2)° and (14.7 ± 0.2)°; or
(4.4 ± 0.2)°, (8.8 ± 0.2)°, (9.2 ± 0.2)°, (12.2 ± 0.2)° and (14.7 ± 0.2)°; or
(4.4 ± 0.2)°, (8.8 ± 0.2)°, (9.2 ± 0.2)°, (12.2 ± 0.2)°, (14.7 ± 0.2)°, and (23.1 ± 0.2)°; or
(4.4 ± 0.2)°, (8.8 ± 0.2)°, (9.2 ± 0.2)°, (12.2 ± 0.2)°, (14.0 ± 0.2)°, (14.7 ± 0.2)°, and (23.1 ± 0.2)°; or
(4.4 ± 0.2)°, (8.8 ± 0.2)°, (9.2 ± 0.2)°, (11.1 ± 0.2)°, (12.2 ± 0.2)°, (14.0 ± 0.2)°, (14.7 ± 0.2)°, and (23.1 ± 0.2)°; or
(4.4 ± 0.2)°, (8.8 ± 0.2)°, (9.2 ± 0.2)°, (11.1 ± 0.2)°, (12.2 ± 0.2)°, (14.0 ± 0.2)°, (14.7 ± 0.2)°, (18.0 ± 0.2)° and (23.1 ± 0.2)°; or
(4.4 ± 0.2)°, (8.8 ± 0.2)°, (9.2 ± 0.2)°, (11.1 ± 0.2)°, (12.2 ± 0.2)°, (14.0 ± 0.2)°, (14.7 ± 0.2)°, (18.0 ± 0.2)°, (21.9 ± 0.2)° and (23.1 ± 0.2)°;
when measured at a temperature in the range of from 20 to 30°C with Cu-Kalpha_{1,2} radiation having a wavelength of 0.15419 nm.

In a further embodiment the invention relates to a crystalline form of voxelotor characterized by having a PXRD comprising reflections at 2-Theta angles of:
(4.4 ± 0.1)°, (8.8 ± 0.1)° and (14.7 ± 0.1)°; or
(4.4 ± 0.1)°, (8.8 ± 0.1)°, (12.2 ± 0.1)° and (14.7 ± 0.1)°; or
(4.4 ± 0.1)°, (8.8 ± 0.1)°, (9.2 ± 0.1)°, (12.2 ± 0.1)° and (14.7 ± 0.1)°; or
(4.4 ± 0.1)°, (8.8 ± 0.1)°, (9.2 ± 0.1)°, (12.2 ± 0.1)°, (14.7 ± 0.1)°, and (23.1 ± 0.1)°; or
(4.4 ± 0.1)°, (8.8 ± 0.1)°, (9.2 ± 0.1)°, (12.2 ± 0.1)°, (14.0 ± 0.1)°, (14.7 ± 0.1)°, and (23.1 ± 0.1)°; or
(4.4 ± 0.1)°, (8.8 ± 0.1)°, (9.2 ± 0.1)°, (11.1 ± 0.1)°, (12.2 ± 0.1)°, (14.0 ± 0.1)°, (14.7 ± 0.1)°, and (23.1 ± 0.1)°; or
(4.4 ± 0.1)°, (8.8 ± 0.1)°, (9.2 ± 0.1)°, (11.1 ± 0.1)°, (12.2 ± 0.1)°, (14.0 ± 0.1)°, (14.7 ± 0.1)°, (18.0 ± 0.1)° and (23.1 ± 0.1)°; or
(4.4 ± 0.1)°, (8.8 ± 0.1)°, (9.2 ± 0.1)°, (11.1 ± 0.1)°, (12.2 ± 0.1)°, (14.0 ± 0.1)°, (14.7 ± 0.1)°, (18.0 ± 0.1)°, (21.9 ± 0.1)° and (23.1 ± 0.1)°;
when measured at a temperature in the range of from 20 to 30°C with Cu-Kalpha_{1,2} radiation having a wavelength of 0.15419 nm.

In another embodiment the present invention relates to a crystalline form of voxelotor characterized by having a PXRD comprising reflections at 2-Theta angles of (8.8 ± 0.2)°, (11.1 ± 0.2)°, (14.0 ± 0.2)°, (14.5 ± 0.2)°, (14.7 ± 0.2)°, (16.0 ± 0.2)°, (20.4 ± 0.2)°, (23.1 ± 0.2)°, (24.9 ± 0.2)° and (25.9 ± 0.2)°, when measured at a temperature in the range of from 20 to 30°C with Cu-Kalpha_{1,2} radiation having a wavelength of 0.15419 nm.

In an additional embodiment the invention relates to a crystalline form of voxelotor characterized by having a PXRD comprising reflections at 2-Theta angles of (8.8 ± 0.1)°, (11.1 ± 0.1)°, (14.0 ± 0.1)°, (14.5 ± 0.1)°, (14.7 ± 0.1)°, (16.0 ± 0.1)°, (20.4 ± 0.1)°, (23.1 ± 0.1)°, (24.9 ± 0.1)° and (25.9 ± 0.1)°, when measured at a temperature in the range of from 20 to 30°C with Cu-Kalpha_{1,2} radiation having a wavelength of 0.15419 nm.

The PXRD of the crystalline form of the present invention can be clearly distinguished from the PXRDs of Form I, Form II and Material N of WO 2015/120133 A1 (see also the PXRD overlay displayed in Figure 2 of the present invention). In particular, the PXRD of the crystalline form of voxelotor of the present invention possesses reflections at (4.4 ± 0.2)°, (8.8 ± 0.2)° and (9.2 ± 0.2)° 2-Theta, whereas no reflections are visible in the PXRDs of Form I, Form II and Material N of WO 2015/120133 A1 in the same ranges. On the other hand, the PXRDs of Form II and Material N show reflections at (5.6° ± 0.2)° 2-Theta and the PXRD of Form I shows a reflection at (5.5° ± 0.2)° 2-Theta, whereas the PXRD of the crystalline form of voxelotor of the present invention displays no reflections in the range of from 5.3 to 5.8° 2-Theta.

Hence, in a preferred embodiment the present invention relates to a crystalline form of voxelotor characterized by having a PXRD as described in any one of the above described embodiments, but not comprising reflections at 2-Theta angles of (5.5 ± 0.2)° and/or (5.6 ± 0.2)°, when measured at a temperature in the range of from 20 to 30°C with Cu-Kalpha_{1,2} radiation having a wavelength of 0.15419 nm.

In another embodiment the present invention relates to a crystalline form of voxelotor characterized by having a PXRD as described in any one of the above described embodiments, but not comprising reflections at 2-Theta angles in the range of from 5.3 to 5.8°, when measured at a temperature in the range of from 20 to 30°C with Cu-Kalpha_{1,2} radiation having a wavelength of 0.15419 nm.

In another embodiment the invention relates to a crystalline form of voxelotor characterized by having a PXRD essentially the same as shown in Figure 1 of the present invention, when measured at a temperature in the range of from 20 to 30°C with Cu-Kalpha_{1,2} radiation having a wavelength of 0.15419 nm.

In yet another embodiment the present invention relates to a crystalline form of voxelotor characterized by having a DSC curve comprising an endothermic peak having an onset at a temperature of (84 ± 5)°C, preferably of (84 ± 3)°C, more preferably of (84 ± 2)°C and most preferably of (84 ± 1)°C, when measured at a heating rate of 10 K/min.

In a further embodiment, the present invention relates to a crystalline form of voxelotor characterized by having a DSC curve comprising an endothermic peak having a maximum at a temperature of (87 ± 5)°C, preferably of (87 ± 3)°C, more preferably of (87 ± 2)°C and most preferably of (87 ± 1)°C, when measured at a heating rate of 10 K/min.

In another aspect the present invention relates to a composition comprising the crystalline form of voxelotor of the present invention as defined in any of the above described embodiments, said composition being essentially free of any other solid-state form of voxelotor. For example, a composition comprising the crystalline form of voxelotor of the present invention comprises at most 20 w-%, preferably at most 10 w-%, more preferably at most 5 w-%, 4 w-%, 3 w-%, 2 w-% or 1 w-% of any other solid-state form of voxelotor, based on the total weight of the composition. Preferably, the any other solid-state form of voxelotor is selected from the group consisting of Form I, Form II and Material N of WO 2015/120133 A1. Form I, Form II and Material N of voxelotor free base all have PXRDs comprising amongst others characteristic reflections at 2-Theta angles of (5.5 ± 0.2)° (Form I) and (5.6 ± 0.2)° (Form II and Material N), respectively. Therefore, the absence of reflections at 2-Theta angles of (5.5 ± 0.2)° and (5.6 ± 0.2)° e.g. the absence of reflections at 2-Theta angles in the range of from 5.3 to 5.8° in the PXRD confirms the absence of voxelotor Form I, Form II and Material N in the composition of the present invention.

Hence, in a preferred embodiment the present invention relates to a composition comprising the crystalline form of voxelotor of the present invention as defined in any of the above described embodiments, characterized by having a PXRD comprising no reflections at 2-Theta angles of (5.5 ± 0.2)° and/or (5.6 ± 0.2)°, when measured at a temperature in the range of from 20 to 30°C with Cu-Kalpha_{1,2} radiation having a wavelength of 0.15419 nm.

In a particular embodiment the present invention relates to a composition comprising the crystalline form of voxelotor of the present invention as defined in any of the above described embodiments, characterized by having a PXRD comprising no reflections at 2-Theta angles in the range of from 5.3 to 5.8°, when measured at a temperature in the range of from 20 to 30°C with Cu-Kalpha_{1,2} radiation having a wavelength of 0.15419 nm.

In a further aspect, the present invention relates to the use of the crystalline form of voxelotor of the present invention or the composition comprising the crystalline form of voxelotor of the present invention as defined in any one of the above described aspects and their corresponding embodiments for the preparation of a pharmaceutical composition.

In a further aspect, the present invention relates to a pharmaceutical composition comprising the crystalline form of voxelotor of the present invention or the composition comprising the crystalline form of voxelotor of the present invention as defined in any one of the above described aspects and their corresponding embodiments, preferably in an effective and/or predetermined amount, and at least one pharmaceutically acceptable excipient.

In one embodiment, the predetermined and/or effective amount of the crystalline form of voxelotor of the present invention is selected from the group consisting of 300 mg, 375 mg, 500 mg, 750 mg and 900 mg calculated as voxelotor. Most preferably, the predetermined and/or effective amount of the crystalline form of voxelotor of the present invention is 500 mg calculated as voxelotor.

In a further embodiment, the pharmaceutical composition of the present invention as described above is an oral solid dosage form. In a particular embodiment, the pharmaceutical composition of the present invention as describe above is a tablet, preferably a film-coated tablet, even more preferably an immediate release film-coated tablet, comprising a tablet core and a coating.

The tablet or tablet core may be prepared by mixing the crystalline form of voxelotor of the present invention with at least one pharmaceutically acceptable excipient followed by compressing the mixture. Optionally, a granulation step such as a wet or dry granulation step is performed before compression. Preferably, the tablet core is subsequently coated with a film-coat. Methods of preparing such tablets, tablet cores and film-coated tablets are well known in the pharmaceutical arts.

In a further aspect, the present invention relates to the crystalline form of voxelotor of the present invention, the composition comprising the crystalline form of voxelotor of the present invention or the pharmaceutical composition comprising the crystalline form of voxelotor of the present invention as defined in any one of the above described aspects and their corresponding embodiments for use as a medicament.

In a further aspect, the present invention relates to the crystalline form of voxelotor of the present invention, the composition comprising the crystalline form of voxelotor of the present invention or the pharmaceutical composition comprising the crystalline form of voxelotor of the present invention as defined in any one of the above described aspects and their corresponding embodiments for use in the treatment of sickle cell disease.

In an alternative embodiment, the invention concerns a method of treating sickle cell disease said method comprising administering an effective amount of the crystalline form of voxelotor of the present invention, the composition comprising the crystalline form of voxelotor of the present invention or the pharmaceutical composition comprising the crystalline form of voxelotor of the present invention as defined in any one of the above described aspects and their corresponding embodiments to a patient in need of such a treatment.

### EXAMPLES

The following non-limiting examples are illustrative for the disclosure and are not to be construed as to be in any way limiting for the scope of the invention.

### Example 1: Preparation of the crystalline form of voxelotor of the present invention

Voxelotor (50 mg, mixture of Form I and Material N, e.g. prepared according to the procedure disclosed in Example 8 of WO 2015/120133 A1) was melted in a glass vial at a temperature ranging from about 97-100°C. The vial containing the melt was then placed into an anodised aluminium block having a temperature of about 95°C. The block was cooled from 95°C to 0°C with a cooling rate of 20K/hour (0.333K/min) and subsequently kept at 0°C for 12 hours. The controlled cooling of the melt yielded the crystalline form of voxelotor of the present invention quantitatively and phase pure.

### Example 2: Powder X-ray diffraction

The crystalline form of voxelotor according to the present invention was investigated by powder X-ray diffraction, which was performed with a PANalytical X'Pert PRO diffractometer equipped with a theta/theta coupled goniometer in transmission geometry, Cu-Kalpha_{1,2} radiation (wavelength 0.15419 nm) with a focusing mirror and a solid state PIXcel detector. The sample was gently ground in an agate mortar with a pestle before the measurement. The diffractogram was recorded at a tube voltage of 45 kV and a tube current of 40 mA, applying a stepsize of 0.013° 2-theta with 40s per step (255 channels) in the angular range of 2° to 40° 2-Theta at ambient conditions. A typical precision of the 2-Theta values is in the range of ± 0.2° 2-Theta, preferably of ± 0.1° 2-Theta.

A representative diffractogram of the crystalline form of voxelotor according to the present invention is displayed in Figure 1 and the corresponding reflection list (peak list) from 2 to 30° 2-Theta is provided in Table 1 below.

**Table 1: Reflection (peak) positions of the crystalline form of voxelotor according to the present invention in the range of from 2 to 30° 2-Theta; A typical precision of the 2-Theta values is in the range of ± 0.2° 2-Theta, preferably of ± 0.1° 2-Theta.**

| **Reflection position [° 2-Theta]** | **Reflection position [° 2-Theta]** | **Reflection position [° 2-Theta]** | **Reflection position [° 2-Theta]** |
|---|---|---|---|
| 4.4 | 14.5 | 19.4 | 24.0 |
| 8.4 | 14.7 | 20.1 | 24.3 |
| 8.8 | 16.0 | 20.4 | 24.9 |
| 9.2 | 16.4 | 21.9 | 25.9 |
| 11.1 | 16.9 | 22.3 | 26.7 |
| 12.2 | 18.0 | 22.9 | 27.0 |
| 13.6 | 18.7 | 23.1 | 28.2 |
| 14.0 | 19.0 | 23.5 | 28.4 |

Figure 2 illustrates an overlay of the PXRDs (from bottom to top) of the crystalline form of voxelotor of the present invention, Form I, Form II and Material N of WO 2015/120133 A1, respectively. As can be seen from this overlay the PXRD of the crystalline form of voxelotor of the present invention can be readily distinguished from the PXRDs of Form I, Form II and Material N of WO 2015/120133 A1. In particular, the PXRD of the crystalline form of voxelotor of the present invention possesses reflections at (4.4 ± 0.2)°, (8.8 ± 0.2)° and (9.2 ± 0.2)° 2-Theta, whereas no reflections are visible in the PXRDs of Form I, Form II and Material N of WO 2015/120133 A1 in the same ranges. On the other hand, the PXRDs of Form II and Material N show reflections at (5.6° ± 0.2)° 2-Theta and the PXRD of Form I shows a reflection at (5.5° ± 0.2)° 2-Theta, whereas the PXRD of the crystalline form of voxelotor of the present invention displays no reflections at all in the range of from 5.3 to 5.8° 2-Theta.

### Example 3: Differential scanning calorimetry (DSC)

The crystalline form of voxelotor of the present invention was investigated by DSC, which was performed on a Mettler Polymer DSC R instrument. The sample (5.41 mg) was heated in a 40 microL aluminium pan with a pierced aluminium lid from 25 to 250°C at a rate of 10 K/min. Nitrogen (purge rate 50 mL/min) was used as purge gas.

The DSC curve shows no thermal event until a first endothermic peak with an onset temperature of about 84°C and a peak temperature of about 87°C appears, which is due to the melting of the sample.

## Claims

1. A crystalline form of voxelotor having the chemical structure as depicted in Formula (I) 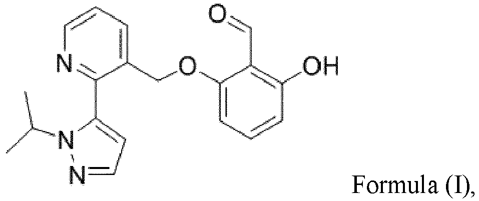 **characterized by** having a powder X-ray diffractogram comprising reflections at 2-Theta angles of (4.4 ± 0.2)°, (8.8 ± 0.2)° and (14.7 ± 0.2)°, when measured at a temperature in the range of from 20 to 30°C with Cu-Kalpha_{1,2} radiation having a wavelength of 0.15419 nm.

2. The crystalline form of claim 1 **characterized by** having a powder X-ray diffractogram comprising additional reflections at 2-Theta angles of (9.2 ± 0.2)° and/or (12.2 ± 0.2)°, when measured at a temperature in the range of from 20 to 30°C with Cu-Kalpha_{1,2} radiation having a wavelength of 0.15419 nm.

3. The crystalline form of claim 1 or 2 **characterized by** having a powder X-ray diffractogram comprising no reflections at 2-Theta angles in the range of from 5.3° to 5.8°, when measured at a temperature in the range of from 20 to 30°C with Cu-Kalpha_{1,2} radiation having a wavelength of 0.15419 nm.

4. The crystalline form according to any one of the preceding claims **characterized by** having a DSC curve comprising an endothermic peak having an onset at a temperature of (84 ± 5)°C, when measured at a heating rate of 10 K/min.

5. A composition comprising the crystalline form as defined in any one of the preceding claims **characterized by** comprising at most 20 weight%, preferably at most 10 weight%, more preferably at most 5 weight%, 4 weight%, 3 weight%, 2 weight% or 1 weight% of any other solid-state form of voxelotor, based on the total weight of the composition.

6. The composition of claim 5 **characterized by** having a powder X-ray diffractogram comprising no reflections at 2-Theta angles in the range of from 5.3 to 5.8°, when measured at a temperature in the range of from 20 to 30°C with Cu-Kalpha1,2 radiation having a wavelength of 0.15419 nm.

7. Use of the crystalline form as defined in any one of claims 1 to 4 or the composition as defined in claim 5 or 6 for the preparation of a pharmaceutical composition.

8. A pharmaceutical composition comprising the crystalline form as defined in any one of claims 1 to 4 or the composition as defined in claim 5 or 6 and at least one pharmaceutically acceptable excipient.

9. The pharmaceutical composition according to claim 8, wherein the pharmaceutical composition is an oral solid dosage form.

10. The pharmaceutical composition of claim 9, wherein the oral solid dosage form is a tablet.

11. The crystalline form as defined in any one of claims 1 to 4, the composition as defined in claim 5 or 6 or the pharmaceutical composition according to any one of claims 8 to 10 for use as a medicament.

12. The crystalline form as defined in any one of claims 1 to 4, the composition as defined in claim 5 or 6 or the pharmaceutical composition according to any one of claims 8 to 10 for use in the treatment of sickle cell disease.
